# EUROPEAN PATENT APPLICATION

(11) **EP 3 865 058 A1**
(43) Date of publication of application: **18.08.2021**
(21) Application number: 18941934.4
(22) Date of filing: 27.11.2018
(51) Int. Cl.: A61B 5/01, A61B 5/00

(54) **CORE BODY TEMPERATURE MEASUREMENT DEVICE HAVING BATTERY CHARGING STRUCTURE**

(30) Priority: 26.11.2018 KR 20180147054
(71) Applicant: Osong Medical Innovation Foundation, Cheongju-si, Chungcheongbuk-do 28160 (KR)
(72) Inventor: JUNG, Ha Chul, Cheongju-si Chungcheongbuk-do 28427 (KR); ROH, Young Hoon, Seoul 06352 (KR); CHOI, Won Jung, Cheongju-si Chungcheongbuk-do 28166 (KR); KIM, Young Jin, Cheongju-si Chungcheongbuk-do 28166 (KR)
(74) Representative: Gaunt, Thomas Derrick
(86) International application number: PCT/KR2018/014719
(87) International publication number: WO 2020/111293

(57) **Abstract**

A core body temperature measurement device includes a fixing frame and a measurement unit. The fixing frame is fixed to an earflap of user. The measurement unit is detachably inserted into the fixing frame and includes an extending part and a temperature sensor. The extending part is bent according to a structure of an ear canal of user and is inserted into the ear canal. The temperature sensor is disposed at an end of the extending part to measure a core body temperature of user.

## Description

### BACKGROUND

### 1. Field of Disclosure

The present disclosure of invention relates to a core body temperature measurement device, and more specifically the present invention relates to a core body temperature measurement device having a battery charging structure, capable of measuring a core body temperature.

### 2. Description of Related Technology

A core body temperature means a temperature of an inside of human body, and a core body temperature measurement device for measuring the core body temperature has been developed variously and widely used.

Conventionally, in the core body temperature, as disclosed by Korean Patent No. 10-1779837, the device is inserted into an ear to measure a temperature of drumhead. Here, a temperature difference between first and second sensors insulated with each other is used to obtain the core body temperature.

In addition, as disclosed by Korean Patent No. 10-1804374, the device is inserted into the ear and the core body temperature is measured using an infrared ray.

However, in the conventional core body temperature device, a sensor is continuously exposed inside of an ear canal, and thus the user may feel uncomfortable or a foreign body. In addition, the exposed portion may be damaged or polluted to be malfunctioned.

Related prior arts are Korean Patent No. 10-1779837 and Korean Patent No. 10-1804374.

### SUMMARY

The present invention is developed to solve the above-mentioned problems of the related arts. The present invention provides a temperature measurement device having a battery charging structure, capable of increasing usage time and being transformed according to a shape or a structure of an ear canal of user to increase convenience of the user.

According to an example embodiment, core body temperature measurement device includes a fixing frame and a measurement unit. The fixing frame is fixed to an earflap of user. The measurement unit is detachably inserted into the fixing frame and includes an extending part and a temperature sensor. The extending part is bent according to a structure of an ear canal of user and is inserted into the ear canal. The temperature sensor is disposed at an end of the extending part to measure a core body temperature of user.

In an example, the fixing frame may include a control part configured to control an operation of the measurement unit, and a power source configured to supply a power to the control part.

In an example, the measurement unit may be inserted into an inserting portion formed at the fixing frame, and the temperature sensor may be electrically connected to the control part as the measurement unit is inserted.

In an example, the measurement unit may further include a distance sensor which is disposed adjacent to the temperature sensor and may be configured to measure a distance toward a drumhead of user. An amount of inserting of the extending part may be determined based on the measurement of the distance sensor.

In an example, the fixing frame may further include a connecting part formed at the inserting portion with a predetermined area. The connecting part may induce an electrical connection between the control part and the temperature sensor, regardless of an inserted length of the extending part.

In an example, the fixing frame may further include a coupling part configured to couple the extending part to the fixing frame, after the amount of the inserting of the extending part is determined.

In an example, the device may further include a fixing cap attached to a front side of the fixing frame and to head for the ear canal of use, so as to limit the inserting of the extending part.

According to the present example embodiments, the fixing frame is fixed to the earflap and the measurement unit is only inserted into the fixing frame to be disposed inside of the ear canal when measuring the temperature, so that the core body temperature measurement device is unnecessary to be always inserted. Thus, the user's convenience may be enhanced.

Here, the control part and the power source are equipped at the fixing frame, and thus the space is enough for receiving the control part and the power source. Thus, the power is stably supplied and the structure of the measurement unit inserted into the ear canal is more simplified.

In addition, the control part is electrically connected when the measurement unit is inserted, so that an additional power connection is unnecessary and the measurement unit is merely operated via the inserting of the measurement unit. Thus, the driving mechanism is more simplified.

In addition, the extending part includes a flexible material and a shape or a structure of the extending part is easily transformed according to the structure of the ear canal, so that the inconvenience of the user may be decreased and the user may feel a foreign body less.

In addition, the extending part is deeply inserted into the ear canal based on the measurement of the distance sensor, and thus the drumhead is prevented from being damaged due to the extending part, and the inserting length of the extending part may be optimally determined by the distance sensor. Further, based on the optimal inserting distance of the extending part, the fixing frame is coupled with the extending part after the optimal inserting of the extending part, so that the core body temperature may be measured more correctly and more reliably.

In addition, according to the optimal inserting length, the position at which the control part and the extending part are electrically connected is changed, so that the connecting part is formed to have a predetermined area, to increase the connecting area between the control part and the extending part. Thus, the connecting between the control part and the extending part may be performed more stably without disconnection, regardless of the inserting length of the extending part.

Alternatively, the fixing cap is attached to the front side of the fixing frame, and the fixing cap includes an elastic material. Thus, using the fixing cap having a relatively simple structure, the extending part is prevented from being inserted into the ear canal over a predetermined distance, so that the drumhead may be prevented from being damage.

### BRIEF DESCRIPTION OF THE DRAWINGS

FIG. 1 is a perspective view illustrating a core body temperature measurement device inserted into an ear canal according to an example embodiment of the present invention;
FIG. 2 is a side view illustrating the core body temperature measurement device of FIG. 1;
FIG. 3 is a schematic view illustrating a core body temperature measurement device inserted into an ear canal according to another example embodiment of the present invention; and
FIG. 4 is a side view illustrating the core body temperature measurement device of FIG. 3.

### * Reference numerals

| | |
|---|---|
| 10, 11 : core body temperature measurement device | 100, 101 : fixing frame |
| 110 : control part | 120 : communication part |
| 130 : power source | 140 : output part |
| 150 : connecting part | 160 : coupling part |
| 200, 201 : measurement unit | 210, 211 : extending part |
| 220 : fixing part | 240 : distance sensor |
| 250, 251 : temperature sensor | 260, 261 : sensor line |
| 270 : power line | 300 : fixing cap |
| 310 : combining part | |

### DETAILED DESCRIPTION

The invention is described more fully hereinafter with Reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art. In the drawings, the size and relative sizes of layers and regions may be exaggerated for clarity.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present invention. Spatially relative terms, such as "beneath," "below," "lower," "above," "upper" and the like, may be used herein for ease of description to describe one element or feature's relationship to another element(s) or feature(s) as illustrated in the figures.

The terminology used herein is for the purpose of describing particular embodiments only and is not intended to be limiting of the invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Unless otherwise defined, all terms (including technical and scientific terms) used herein have the same meaning as commonly understood by one of ordinary skill in the art to which this invention belongs.

It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The invention is described more fully hereinafter with reference to the accompanying drawings, in which embodiments of the invention are shown.

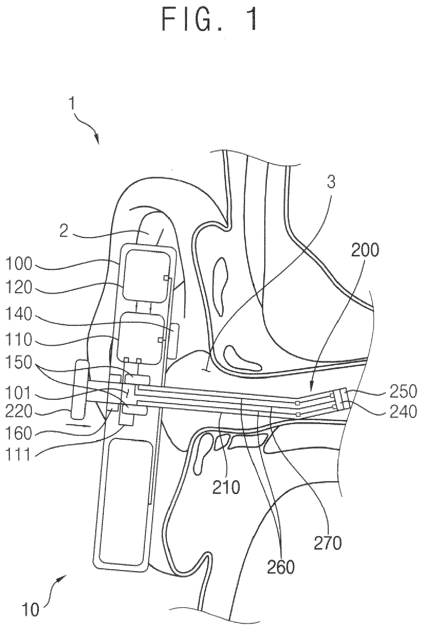

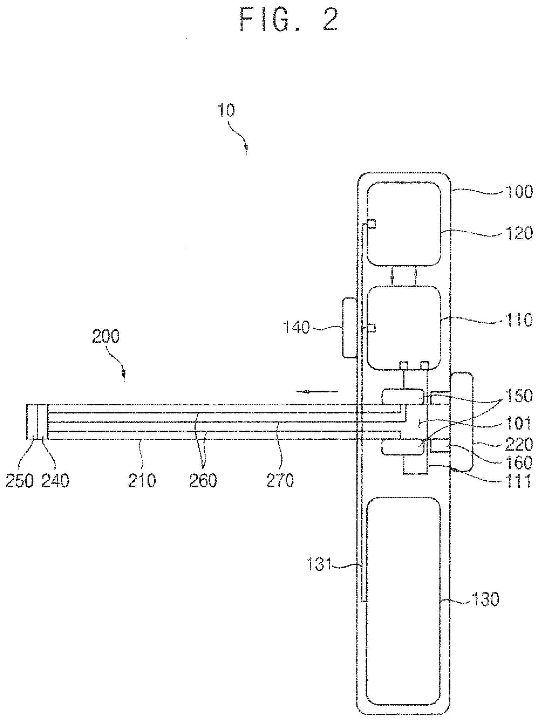

FIG. 1 is a perspective view illustrating a core body temperature measurement device inserted into an ear canal according to an example embodiment of the present invention. FIG. 2 is a side view illustrating the core body temperature measurement device of FIG. 1.

Referring to FIG. 1 and FIG. 2, the core body temperature measurement device 10 according to the present example embodiment is fixed at an earflap 2 of an ear 1 of user, and is inserted into an ear canal 3, to measure a core body temperature of the user. The core body temperature measurement device 10 includes a fixing frame 100 and a measurement unit 200.

The fixing frame 100 is fixed to the earflap 2 of the user. To be fixed to the earflap 2, although not shown in the figure, an additional fixing part may be connected to the fixing frame to increase a fixing force with the earflap 2.

In addition, in the figure, a cross-sectional shape of the fixing frame 100 is a longitudinal rectangular shape, but the shape of the fixing frame 100 is not limited thereto if the fixing frame 100 is fixed at the earflap 2. For example, the fixing frame 100 may have a circular or elliptic plate shape having a predetermined thickness.

The fixing frame 100 is not inserted into an inside of the ear canal 3, and thus an inner space of the fixing frame 100 may be large enough to receive various elements. Thus, the fixing frame 100 includes a control part 110, a communication part 120, a power source 130, an output part 140, a connecting part 150 and a coupling part 160 which are disposed in the inner space of the fixing frame 100.

In FIG. 1 and FIG. 2, the measurement unit 200 is illustrated to be inserted, but an inserting portion 101 through which the measurement unit 200 is inserted is formed through a central portion of the fixing frame 100.

The control part 110 controls the measurement unit 200 and also controls an overall operation of the device 10. The control part 110 starts to control when the measurement unit 200 is inserted into the fixing frame 100 and the measurement unit 200 is electrically connected.

The control part 110 controls the operation of sensors of the measurement unit 200, controls the operation of the power source 130 and the output part 140, and further controls the control operation via receiving the power from the power source 130.

In addition, the control part 110 controls the coupling part 160, so that the extending part 210 is coupled with the fixing frame 100 by the coupling part 160.

The communication part 120 provides control information or stored information of the control part 100 to outside, or provides the received information to the control part 110. The communication part 120 may be a wireless network module such as a Bluetooth network module.

The power source 130 may be controlled by the control part 110, and provides the power for the operation of the control part 110, the communication part 120, the coupling part 160 and the measurement unit 200.

Here, the power source 130 may be a rechargeable battery, and thus may receive the power from outside for the charging.

The output part 140 may be disposed inside of the fixing frame 100, but alternatively, as illustrated in the figure, the output part 140 may be disposed at an outside of the fixing frame to head for the ear canal 3.

The output part 140 may be, for example, a speaker, and thus informs the operation state of the device 10 to the user. In addition, the output part 140 may also provide the information on the safety, such as a distance between the extending part and the drumhead of the user, as explained below.

The connecting part 150 is disposed around the inserting portion 101, or may be disposed at a circumference of the inserting portion 101 when the inserting portion 101 has a cylindrical shape. The connecting part 150 is electrically connected with the control part 110 via a connecting line 111.

Here, the connecting part 150 is formed to have a predetermined distance along an extending direction of the inserting portion 101 which is an inserting direction of the measurement unit 200, and thus, the connecting part 150 has a predetermined area according to a circumferential surface of the inserting portion 101.

A surface of the connecting part 150 facing the inserting portion 101 is exposed to be capable of being electrically connected.

The coupling part 160 is also disposed along the circumferential surface of the inserting portion 101, and the coupling part 160 is spaced apart from the connecting part 150. Thus, the coupling part 160 is not electrically connected with the connecting part 150, and is only controlled by the control part 110.

The coupling part 160 is operated by the driving of the control part 110. Thus, as the coupling part 160 is driven to be operated, the measurement unit 200 is fixed to the inserting portion 101, but as the coupling part 160 is released or decoupled by the control part 110, the fixing force between the measurement unit 200 and the inserting portion 101 is released.

Here, the coupling part 160 may be an electromagnet. Thus, as the control of the control part 110, the electromagnet is operated to fix the measurement unit 200 to the inserting portion 101 as a current is supplied to the coupling part 160. However, the measurement unit 200 is detached from the inserting portion 101 as the current is stopped to be provided to the coupling part 160.

The measurement unit 200 is inserted into or detached from the fixing frame 100, and includes an extending part 210, a fixing part 220, a distance sensor 240, a temperature sensor 250, a sensor line 260 and a power line 270. The measurement unit 200 has a 'T' shape.

The extending part 210 extends along a direction, and is inserted into the inserting portion 101. Thus, the extending part 210 is inserted into the ear canal 3 of the user.

Here, a shape or a structure of an inside of the ear canal 3 is different for each user, and a length to the drumhead is also different for each user.

Thus, in the present example embodiment, the extending part 210 includes a flexible material and is capable of being bent. Thus, as illustrated in FIG. 1, an end of the extending part 210 may be bent and extended according to the structure or the shape of the ear canal 3 of the user. Thus, the user may feel inconvenience or a foreign body less.

The fixing part 220 is connected to an end of the extending part 210. The fixing part 200 makes contact with an outer surface of the fixing frame 100 to block an additional inserting of the extending part 210, when the extending part 210 is inserted over a predetermined distance. Here, a diameter of the fixing part 220 is larger than an inner diameter of the inserting portion 101.

The fixing part 220 is connected to the end of the extending part 210, and thus as illustrated as an arrow in FIG. 2, the inserting direction of the extending part 210 is from the end (a first end) connected with the fixing part 220 to an opposite end (a second end) of the extending part 210.

The temperature sensor 250 is disposed at the second end of the extending part 210, and measures the core body temperature of the user when the extending part 210 is inserted into the ear canal 3.

The temperature sensor 250 may be, for example, a thermistor, and is electrically connected to the connecting part 150 via the sensor line 260. Thus, the temperature sensor 250 is controlled by the control part 110, and receives the power from the power source 130.

A position of the sensor line 260 is not changeable, and the sensor line 260 is exposed and fixed at the predetermined position. As explained above, the position of the sensor line 260 on the inserting portion 101 may be changed according the inserting length of the extending part 210.

However, in the present example embodiment, the connecting part 150 having the predetermined area is formed on the inserting portion 101 and is exposed to the inserting portion 101, and thus the sensor line 260 is stably and electrically connected with the connecting part 150 within a predetermined distance range, even though the position of the sensor line 260 is changed as explained above.

The distance sensor 240 is disposed at the second end of the extending part 210 and is disposed adjacent to the temperature sensor 250. The distance sensor 240 measures a distance between the second end of the temperature sensor 250 and the drumhead, as the extending part 210 is inserted into the ear canal 3.

Here, as illustrated in FIG. 2, the temperature sensor 250 is disposed at the very end of the extending part 210, but the positions of the temperature sensor 250 and the distance sensor 240 may be changed with each other.

The distance sensor 240 may be electrically connected with the connecting part 150 via the power line 270, and thus the distance sensor 240 may be controlled by the control part 110. In addition, the distance sensor 240 may also receive the power from the power source 130.

Here, the electrical connection between the distance sensor 240 and the connecting part 150 is stably performed same as explained the electrical connection between the temperature sensor 250 and the connecting part 150.

Here, as illustrated in the figure, the distance sensor 240 and the temperature sensor 250 are electrically connected with the same connecting part 150, but alternatively, the connecting portions or the connecting areas are divided such that the distance sensor 240 is electrically connected with a first area of the connecting part 150 and the temperature sensor 250 is electrically connected with a second area thereof different from the first area thereof.

However, even though the connecting portions are divided, the connecting part 150 has the predetermined connecting area and thus the connection may be stably performed regardless of an amount of the inserting of the extending part 210.

The control part 110 decides whether the distance between the extending part 210 and the drumhead which is measured by the distance sensor 240 is less than a predetermined minimum maintenance distance. Here, the control part 110 already received the predetermined minimum maintenance distance and then decides. The predetermined minimum maintenance distance may be defined as a minimum distance between the second end of extending part 210 and the drumhead in which the drumhead is not damaged by the extending part 210.

The control part 110 outputs an alarm or a warning message using the output part 140 and blocks the extending part 210 from being inserted additionally, when the distance between the extending part 210 and the drumhead is less than the predetermined minimum maintenance distance.

When the distance between the extending part 210 and the drumhead, which is measured by the distance sensor 240, reaches a predetermined optimal distance, the control part 110 provides an alarm via the output part 140, to inform the inserting position of the extending part 210. Further, the control part 110 operates the coupling part 160 to fix the extending part 210 to the fixing frame 100, so that the inserting position of the extending part 210 is fixed when the extending part 210 reaches the predetermined optimal distance.

Here, the predetermined optimal distance may be a distance between the predetermined minimum maintenance distance and a predetermined minimum measurable distance. The core body temperature may be measured over the predetermined minimum measurable distance.

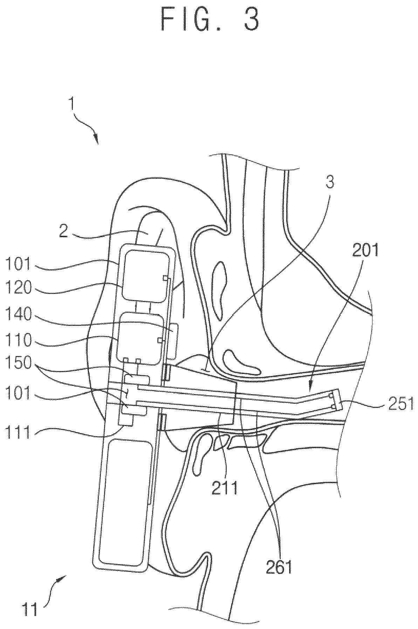

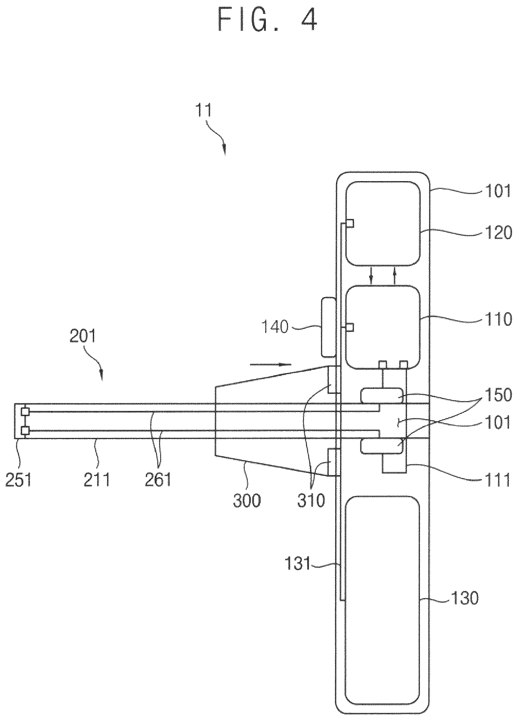

FIG. 3 is a schematic view illustrating a core body temperature measurement device inserted into an ear canal according to another example embodiment of the present invention. FIG. 4 is a side view illustrating the core body temperature measurement device of FIG. 3.

The core body temperature measurement device according to the present example embodiment is substantially same as the core body temperature device in FIG. 1 and FIG. 2, except that a fixing cap and a combining part are further included and the fixing part and the coupling part are excluded, and thus same reference numerals are used for same elements and any repetitive explanation will be omitted.

Referring to FIG. 3 and FIG. 4, the core body temperature measurement device according to the present example embodiment includes the fixing frame 101 and the measurement unit 201, and further includes the fixing cap 300 and the combining part 310.

The fixing frame 101 has the inserting portion 101 at the center thereof, and the measurement unit 201 is inserted into the inserting portion 101. In the present example embodiment, the measurement unit 201 is inserted into and fixed to the inserting portion 101 along the illustrated direction, according to the structure of the fixing cap 300.

In the present example embodiment, the measurement unit 201 includes the temperature sensor 251 and the sensor line 261 electrically connecting the temperature sensor 251 with the connecting part 150, and the distance sensor and the power line may be omitted.

In the previous example embodiment in FIG. 1, the safety distance between the extending part 211 and the drumhead is maintained using the distance sensor, but in the present example embodiment, the fixing cap 300 prevents the extending part 211 from being inserted into the ear canal 3 over the predetermined distance and the drumhead is prevented from being damage.

The fixing cap 300 is fixed at a front side of the fixing frame 101, and the extending part 211heading for the inside of the ear canal 3 passes through the fixing cap 300 and is fixed to the fixing cap 300. The combining part 310 is disposed between the fixing cap 300 and the fixing frame 101. Here, the combining part 310 is disposed at a contact portion between the fixing cap 300 and the fixing frame 101.

Here, the combining part 310 may be, for example, a magnet, and thus the fixing cap 300 and the fixing frame 101 are combined with each other by a magnetic force.

The extending part 211 passes through the center of the fixing cap 300, and the inner surface of the fixing cap 300 makes tight contact with the outer surface of the extending part 211. A radius of the fixing cap 300 is decreased as the fixing cap 300 closes to the inside of the ear canal 3, and thus the cross-sectional shape of the fixing cap 300 is a trapezoidal shape.

Thus, the fixing cap 300 is prevented from being inserted into the inside of the ear canal 3 over the predetermined depth, and thus the extending part 211 is also limited to be inserted into the inside of the ear canal 3 over the predetermined depth.

Thus, even though the distance sensor is omitted, the extending part 211 is prevented from being inserted into the ear canal 3 over the predetermined depth, and thus the drumhead is prevented from being damaged.

Further, the change rate of the radius or the radius of the fixing cap 300 may be variously selected, to form the fixing cap 300 with a various shape. Thus, the fixing cap 300 may be changed according to the user's age or gender and so on, to control the inserting depth or distance of the extending part 211 properly.

Here, based on the measured distance or depth of the ear canal for each user, the optimal fixing cap 300 may be selected, so that each user may use the core body temperature measurement device 11 having the optimal fixing cap 300, for measuring the core body temperature. Thus, the convenience and the safety for each user may be more increased.

According to the present example embodiments, the fixing frame is fixed to the earflap and the measurement unit is only inserted into the fixing frame to be disposed inside of the ear canal when measuring the temperature, so that the core body temperature measurement device is unnecessary to be always inserted. Thus, the user's convenience may be enhanced.

Here, the control part and the power source are equipped at the fixing frame, and thus the space is enough for receiving the control part and the power source. Thus, the power is stably supplied and the structure of the measurement unit inserted into the ear canal is more simplified.

In addition, the control part is electrically connected when the measurement unit is inserted, so that an additional power connection is unnecessary and the measurement unit is merely operated via the inserting of the measurement unit. Thus, the driving mechanism is more simplified.

In addition, the extending part includes a flexible material and a shape or a structure of the extending part is easily transformed according to the structure of the ear canal, so that the inconvenience of the user may be decreased and the user may feel a foreign body less.

In addition, the extending part is deeply inserted into the ear canal based on the measurement of the distance sensor, and thus the drumhead is prevented from being damaged due to the extending part, and the inserting length of the extending part may be optimally determined by the distance sensor. Further, based on the optimal inserting distance of the extending part, the fixing frame is coupled with the extending part after the optimal inserting of the extending part, so that the core body temperature may be measured more correctly and more reliably.

In addition, according to the optimal inserting length, the position at which the control part and the extending part are electrically connected is changed, so that the connecting part is formed to have a predetermined area, to increase the connecting area between the control part and the extending part. Thus, the connecting between the control part and the extending part may be performed more stably without disconnection, regardless of the inserting length of the extending part.

Alternatively, the fixing cap is attached to the front side of the fixing frame, and the fixing cap includes an elastic material. Thus, using the fixing cap having a relatively simple structure, the extending part is prevented from being inserted into the ear canal over a predetermined distance, so that the drumhead may be prevented from being damage.

Although the exemplary embodiments of the present invention have been described, it is understood that the present invention should not be limited to these exemplary embodiments but various changes and modifications can be made by one ordinary skilled in the art within the spirit and scope of the present invention as hereinafter claimed.

## Claims

1. A core body temperature measurement device comprising:
a fixing frame fixed to an earflap of user; and
a measurement unit detachably inserted into the fixing frame and comprising an extending part and a temperature sensor,
wherein the extending part is bent according to a structure of an ear canal of user and is inserted into the ear canal,
wherein the temperature sensor disposed at an end of the extending part to measure a core body temperature of user.

2. The device of claim 1, wherein the fixing frame comprises:
a control part configured to control an operation of the measurement unit; and
a power source configured to supply a power to the control part.

3. The device of claim 2, wherein the measurement unit is inserted into an inserting portion formed at the fixing frame, and the temperature sensor is electrically connected to the control part as the measurement unit is inserted.

4. The device of claim, 3 wherein the measurement unit further comprises a distance sensor which is disposed adjacent to the temperature sensor and is configured to measure a distance toward a drumhead of user,
wherein an amount of inserting of the extending part is determined based on the measurement of the distance sensor.

5. The device of claim 4, wherein the fixing frame further comprises a connecting part formed at the inserting portion with a predetermined area,
wherein the connecting part induces an electrical connection between the control part and the temperature sensor, regardless of an inserted length of the extending part.

6. The device of claim 4, wherein the fixing frame further comprises a coupling part configured to couple the extending part to the fixing frame, after the amount of the inserting of the extending part is determined.

7. The device of claim 1, further comprising:
a fixing cap attached to a front side of the fixing frame and to head for the ear canal of use, so as to limit the inserting of the extending part.
